(19) **Europäisches Patentamt / European Patent Office / Office européen des brevets**

(11) **EP 3 691 614 B1**

(12) **EUROPEAN PATENT SPECIFICATION**

(45) Date of publication and mention of the grant of the patent:
**01.11.2023   Bulletin 2023/44**

(21) Application number: **18788663.5**

(22) Date of filing: **01.10.2018**

(51) International Patent Classification (IPC):
**A61K 9/20** *(2006.01)*      **A61K 31/40** *(2006.01)*

(52) Cooperative Patent Classification (CPC):
**A61K 9/2054; A61K 9/2027; A61K 9/2059; A61K 31/40; A61P 3/10**

(86) International application number:
**PCT/EP2018/025254**

(87) International publication number:
**WO 2019/068367 (11.04.2019 Gazette 2019/15)**

(54) **PHARMACEUTICAL COMPOSITION COMPRISING VILDAGLIPTIN AND METHOD OF PREPARATION THEREOF**

PHARMAZEUTISCHE ZUSAMMENSETZUNG MIT VILDAGLIPTIN UND VERFAHREN ZUR HERSTELLUNG DAVON

COMPOSITION PHARMACEUTIQUE COMPRENANT DE LA VILDAGLIPTINE ET SA MÉTHODE DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.10.2017   GR 20170100466**

(43) Date of publication of application:
**12.08.2020   Bulletin 2020/33**

(73) Proprietor: **Pharmathen S.A.**
**15351 Pallini Attikis (GR)**

(72) Inventors:
• **KARAVAS, Evangelos**
  **153 51 Pallini Attikis (GR)**
• **KOUTRIS, Efthymios**
  **153 51 Pallini Attikis (GR)**
• **SAMARA, Vasiliki**
  **153 51 Pallini Attikis (GR)**
• **KOUTRI, Ionna**
  **153 51 Pallini Attikis (GR)**
• **KALASKANI, Anastasia**
  **153 51 Pallini Attikis (GR)**
• **KAKOURIS, Andreas**
  **153 51 Pallini Attikis (GR)**
• **MPENEKIS, Vasilis**
  **153 51 Pallini Attikis (GR)**

(56) References cited:
**WO-A1-2018/050892      WO-A2-2006/078593**

Remarks:
The file contains technical information submitted after the application was filed and not included in this specification

**Description**

TECHNICAL FIELD OF THE INVENTION

The present invention relates to a solid pharmaceutical formulation of Vildagliptin or a pharmaceutically acceptable salt thereof. The main objective of the present invention is to provide a formulation of Vildagliptin that is stable and robust and the manufacturing process is easy and cost effective.

BACKROUND OF THE INVENTION

Diabetes mellitus is a common disorder more prevalent in developed countries. It is a metabolic disease wherein an individual has high blood sugar level over a prolonged period of time. There are three types of the disease. In Type 1 diabetes the pancreas fails to produce enough insulin, therefore it requires immediate and life-long treatment with insulin. Type 2 diabetes is a chronic and progressive disease arising from a complex pathophysiology involving the dual endocrine defects of insulin resistance and impaired insulin secretion. The treatment typically begins with diet and exercise; however, it will be followed by oral antidiabetic monotherapy, before moving into a combination regime, because most patients find it hard to sufficiently control glycaemia during long-term treatment with diet and exercise alone. And the third form of the disease is gestational diabetes, which occurs when pregnant women develop high blood sugar levels and is treated with diet and exercise and in some cases insulin too.

Drugs of choice for therapy include biguanides, Dipeptidyl peptidase-IV (DPP-IV) inhibitors, sulfonylurea, thiazolidinedione, alphaglucosidase inhibitor, amylin analog, glucagon-like peptide-1 (GLP-1) or incretin mimetic, meglitinide and insulin. DPP-IV inhibitors represent a class of agents that are being developed for the treatment or improvement in glycemic control in patients with type 2 diabetes. More specifically, Vildagliptin, also known as LAF-237 is the generic name for (S)-1-[(3-hydroxy-1-adamantyl)amino]acetyl-2-cyano-pyrrolidine and has been disclosed specifically in US patent no 6,166,063; it was introduced in 2006, it is easy to use and does not require regular glucose monitoring or dose adjustments. Vildagliptin has been found to reduce fasting glucose and postprandial glucose excursion in association with significantly reduced $HbA_{1c}$ levels.

It is well known in the art that DPP-IV inhibitors with primary or secondary amino group show incompatibilities, degradation problems or extraction problems with some excipients especially excipients that have acidic properties. Vildagliptin has also a secondary amino group on its chemical structure. In solid dosage forms, it may react with many excipients or impurities of excipients. Although vildagliptin itself is very stable it has high susceptibility to air and humidity. This fact causes impurities to occur in the composition and leads to incorporation of undesired components into the composition. EP 2468361 discloses a pharmaceutical composition, comprising vildagliptin granules which are coated with at least one coating layer and one or more than one excipients. In patent applications EP1841413 and EP2165703, direct compression is used to develop tablet formulation of DPP-IV inhibitor compounds, especially vildagliptin or an acid addition salt thereof.

Vildagliptin is marketed under the trademark Galvus® in 50 mg dosage forms by Novartis. It is indicated for the treatment of diabetes mellitus, but particularly in treating type 2 diabetes. Galvus® includes lactose anhydrose, microcrystalline cellulose, sodium starch glycolate and magnesium stearate.

The present invention relates to tablets, especially tablets formed by direct compression of a DPP-4 inhibitor compound, and a process for preparation thereof.

SUMMARY OF THE INVENTION

The present invention is defined by claims 1-8.

It is, therefore, an object of the present invention to provide a pharmaceutical formulation comprising Vildagliptin or a pharmaceutically acceptable salt thereof formed by direct compression.

A further object of the present invention is to provide a manufacturing process that is easy and cost effective and overcomes the problems of impurities due to reaction of Vildagliptin to excipients present in the formulation.

Another aspect of the present invention is to provide a pharmaceutical formulation comprising Vildagliptin or pharmaceutically acceptable salt thereof, wherein the selection of excipients has been optimized and the production of impurities in the formulation has been minimized.

The main objective of the present invention is to provide a proper qualitative and quantitative composition of Vildagliptin or pharmaceutical acceptable salt thereof, prepared by a suitable manufacturing process in order to obtain a stable and efficacious dosage form with good physicochemical characteristics.

Another object of the present invention is to provide a pharmaceutical formulation comprising Vildagliptin or a pharmaceutically acceptable salt thereof and further comprising an appropriate amount of a disintegrant, wherein the disintegrant doesn't react with the active agent, and wherein the disintegrant is croscarmellose sodium.

According to the present invention, a process for the preparation of a stable dosage form of Vildagliptin is provided, comprising an effective amount of a disintegrant and a combination of diluents, and a lubricant, which process comprises the steps of: blending the active agent with the disintegrant and the diluents, adding the lubricant and compressing into tablets.

Other objects and advantages of the present invention will become apparent to those skilled in the art in view of the following detailed description.

DETAILED DESCRIPTION OF THE INVENTION

There are three commercially important processes for making compressed tablets: wet granulation, direct compression and dry granulation (slugging or roller compaction). The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets. Preformulation studies are done to determine the chemical and physical compatibility of the active component with proposed excipients.

The properties of the drug, its dosage forms and the economics of the operation will determine selection of the best process for tableting. Generally, both wet granulation and direct compression are used in developing a tablet.

The dry granulation method may be used where one of the constituents, either the drug or the diluent, has sufficient cohesive properties to be tabletted. The method consists of blending, slugging the ingredients, dry screening, lubrication and compression.

The wet granulation method is used to convert a powder mixture into granules having suitable flow and cohesive properties for tableting. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

In general, powders do not have sufficient adhesive or cohesive properties to form hard, strong granules. A binder is usually required to bond the powder particles together due to the poor cohesive properties of most powders. Heat and moisture sensitive drugs cannot usually be manufactured using wet granulation. The large number of processing steps and processing time are problems due to high level manufacturing costs. Wet granulation has also been known to reduce the compressibility of some pharmaceutical excipients, such as microcrystalline cellulose.

Direct compression is regarded as a relatively quick process where the powdered materials are compressed directly without changing the physical and chemical properties of the drug. The active ingredient(s), direct compression excipients and other auxiliary substances, such as a glidant and lubricant are blended in a twin shell blender or similar low shear apparatus before being compressed into tablets. This type of mixing was believed to be essential in order to prepare "pharmaceutically acceptable" dosage forms. Some pharmaceutical scientists believe that the manner in which a lubricant is added to a formulation must be carefully controlled. Accordingly, lubricants are usually added to a granulation by gentle mixing. It is also believed that prolonged blending of a lubricant with a granulation can materially affect hardness and disintegration time for the resulting tablets. Excessive blending of lubricants with the granulate ingredients can cause water proofing of the granule and reduces tablet hardness or strength of the compressed tablet. For these reasons, high-shear mixing conditions have not been used to prepare direct compression dosage forms.

The advantages of direct compression include uniformity of blend, few manufacturing steps involved, i.e., the overall process involves weighing of powders, blending and compression, hence less cost; elimination of heat and moisture, prime particle dissociation and physical stability.

Pharmaceutical manufacturers would prefer to use direct compression techniques over wet or dry granulation methods because of quick processing time and cost advantages. However, direct compression is usually limited to those situations where the drug or active ingredient has physical characteristics required to form pharmaceutically acceptable tablets. However, one or more excipients must often be combined with the active ingredient before the direct-compression method can be used since many ingredients do not have the necessary properties. Since each excipient added to the formulation increases the tablet size of the final product, manufacturers are often limited to using the direct-compression method in formulations containing a low dose of the active ingredient per compressed tablet.

A solid dosage form containing a high-dose drug, i.e., the drug itself comprises a substantial portion of the total compressed tablet weight, could only be directly compressed if the drug itself has sufficient physical characteristics, e.g., cohesiveness, for the ingredients to be directly compressed.

For example, DPP-IV inhibitors are considered high- dose drugs. Most tablet formulations include a range of 70-85% by weight of DPP-IV inhibitor per tablet. This high-dose drug, combined with its rather poor physical characteristics for direct compression, has not permitted direct compression as a method to prepare the final tablet. In addition, the active ingredients have poor stability in presence of water, another factor militating against the use of the wet granulation method.

Another limitation of direct compression as a method of tablet manufacturing is the potential size of the compressed tablets. If the amount of active ingredient is high, a pharmaceutical formulator may choose to wet granulate the active ingredient with other excipients to attain an acceptable sized tablet with the desired amount of active ingredient. The amount of filler, binder or other excipients needed in wet granulation is less than that required for direct compression since the process of wet granulation contributes toward the desired physical properties of the tablet.

In spite of the advantages afforded by wet granulation in general, due to the instability of the compounds in the presence of water, it is desirable to directly compress tablets containing high-dose DPP-IV inhibitors. There is a need in the industry for techniques and pharmaceutical excipients which will allow manufacturers to prepare high-dose DPP-IV inhibitor tablets by direct compression,

The present invention is directed to a pharmaceutical composition comprising Vildagliptin or a pharmaceutically acceptable salt thereof as the pharmaceutically active ingredient and at least one disintegrant, wherein 90% of the particles by volume of the active pharmaceutical ingredient is between 350 and 450 μm as measured by laser light diffraction, wherein the amount of the disintegrant is from 7 to 9 % by weight of the composition.

The present invention is also directed to a process for the preparation of a pharmaceutical composition according to claim 1 comprising the steps of:

- Mixing Vildagliptin or a pharmaceutically acceptable salt thereof having 90% of the particles by volume between 350 and 450 μm as measured by laser light diffraction with at least one disintegrant in the amount of from 7 to 9 % by weight of the composition and optionally other pharmaceutically acceptable excipient until a uniform blend is reached;
- Adding to the above mixture a pharmaceutically acceptable glidant and mixing until uniformity of blend;
- Compressing the resulting mixture into tablets;
- Optionally coating the tablets.

It is an object of the invention to provide a Vildaglitpin formulation in the form of a free-flowing, cohesive tableting powder, capable of being directly compressed into a tablet.

It is a further object of the invention to provide a direct compressed Vildagliptin in a unit dosage form having an acceptable dissolution profile, as well as acceptable degrees of hardness and resistance to chipping, as well as a short disintegration time. It is a further object of the invention to provide a process for preparing a compressed Vildagliptin tablet by direct compression in unit dosage form.

The present invention provides a direct tableting, free-flowing particulate Vildagliptin formulation in the form of a tableting powder, capable of being directly compressed into a tablet having adequate hardness, rapid disintegration time and an acceptable dissolution pattern.

Hydroxypropyl methylcellulose has been utilized in the pharmaceutical industry as a direct compression excipient for solid dose forms. Hydroxypropyl methylcellulose is a processed cellulose and controls drug release from solid dosage forms.

In addition to the active ingredient, the tableting powder contains a number of inert materials known as excipients. They may be classified according to the role they play in the final tablet. The primary composition includes fillers, binders or diluents, lubricants, disintegrants and glidants. Other excipients which give physical characteristics to the finished tablet are coloring agents, and flavors in the case of chewable tablets. Typically, excipients are added to a formulation to impart good flow and compression characteristics to the material being compressed.

The preferred formulation of this invention comprises the following: Vildagliptin as the active ingredient, the binders or diluents which are microcrystalline cellulose and lactose, the disintegrant which is croscarmellose sodium and the lubricant which is magnesium stearate. The excipients have been carefully selected after many optimization steps.

One, two, three or more diluents can be selected. Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 15% to about 40% by weight of the composition. The preferred diluents include microcrystalline cellulose which is manufactured by the controlled hydrolysis of alpha-cellulose, obtained as a pulp from fibrous plant materials, with dilute mineral acid solutions. Following hydrolysis, the hydrocellulose is purified by filtration and the aqueous slurry is spray dried to form dry, porous particles of a broad size distribution. Suitable microcrystalline cellulose will have an average particle size of from about 20 nm to about 200 nm. Microcrystalline cellulose is available from several suppliers. Suitable microcrystalline cellulose includes Avicel PH 101, Avicel PH 102, Avicel PH 103, Avicel PH 105 and Avicel PH 200, manufactured by FMC Corporation. Particularly preferred in the practice of this invention is Avicel PH 102, which has the smallest surface area and pore structure. Preferably the microcrystalline cellulose is present in a tablet formulation in an amount of from about 25% to about 70% by weight, more preferably from about 30% to about 60% by weight; and most preferably from about 40% to about 50% by weight.

Another diluent is lactose. Preferably, the lactose is ground to have an average particle size of between about 50 μm and about 500 μm prior to formulating. The lactose is present in the tablet formulation in an amount preferably from about 5% to about 40% by weight, more preferably from about 18% to about 35% by weight, and most preferred from about 20% to about 25% by weight.

A disintegrant is a component of the tablet formulation. Disintegrants are included to ensure that the tablet has an acceptable rate of disintegration. One, two, three or more disintegrants can be selected. Examples of pharmaceutically acceptable disintegrants include, but are not limited to, starches; clays; celluloses; alginates; gums; cross-linked polymers, e.g., cross-linked polyvinyl pyrrolidone, cross-linked calcium carboxymethylcellulose and cross-linked sodium carboxymethylcellulose (croscarmellose sodium); soy polysaccharides; and guar gum, sodium starch glycolate. The disintegrant is present in an amount from 7% to 9% by weight of the composition.

Lubricants are typically added to prevent the tableting materials from sticking to punches, minimize friction during tablet compression and allow for removal of the compressed tablet from the die. Such lubricants are commonly included in the final tablet mix in amounts usually less than 1% by weight. The lubricant component may be hydrophobic or hydrophilic. One, two, three or more lubricants can be selected. Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant may be present in an amount preferably from about 0.1% to about 5% by weight of the composition and most preferably from about 0.1% to about 2% by weight of the composition. Magnesium stearate was the choice for the present invention. It reduces the friction between the die wall and tablet mix during the compression and ejection of the tablets. It helps prevent adhesion of tablets to the punches and dies. Magnesium stearate also aids in the flow of the powder in the hopper and into the die. It has a particle size range of 450-550 microns and a density range of 1.00-1.80 g/mL. It is stable and does not polymerize within the tableting mix.

Other conventional solid fillers or carriers, such as, cornstarch, calcium phosphate, calcium sulfate, calcium stearate, magnesium stearate, stearic acid, glyceryl mono- and distearate, sorbitol, mannitol, gelatin, natural or synthetic gums, such as carboxymethyl cellulose, methyl cellulose, alginate, dextran, acacia gum, karaya gum, locust bean gum, tragacanth and the like, diluents, binders, lubricants, coloring and flavoring agents could optionally be employed.

Examples of pharmaceutically acceptable binders include, but are not limited to, starches; celluloses and derivatives thereof, e.g., microcrystalline cellulose, hydroxypropyl cellulose hydroxylethyl cellulose and hydroxylpropylmethyl cellulose; sucrose; dextrose; corn syrup; polysaccharides; and gelatin. The binder, e.g., may be present in an amount from about 10% to about 40% by weight of the composition.

Additional examples of useful excipients are described in the Handbook of pharmaceutical excipients, 3rd edition, published by: American Pharmaceutical Association, or Handbook of Pharmaceutical Excipients, 4th edition, published by Science and Practice.

Composition as described above, wherein at least one diluent is a microcrystalline cellulose and wherein in the unit dosage form, the weight of Vildagliptin on a dry weight basis to tablet weight of microcrystalline cellulose ratio is of 1 to 3, preferably 1 to 2, most preferably of 1 to 1.80.

Compositions as described above, wherein the weight ratio between diluent or combination of diluents and disintegrant, when the diluent is selected from lactose and/or microcrystalline cellulose and the disintegrant is selected from croscarmellose sodium is from 5:1, preferably from 7.5:1, more preferably is 10:1.

Composition as described above comprising between 20 and 70 mg Vildagliptin or a pharmaceutically acceptable salt thereof, preferably 50 mg Vildagliptin or a pharmaceutically acceptable salt thereof.

Composition as described above wherein the diluent is selected from a microcrystalline cellulose and lactose, preferably microcrystalline cellulose and lactose are in the composition. Composition as described above, wherein the disintegrant is selected from croscarmellose sodium.

Finally, the inventors have also concluded that for optimization of the directly compressed Vildagliptin tablet the particle size of the particles comprising Vildagliptin in the tablet should have 90% of the particle size distribution of the particles by volume comprising Vildagliptin in the tablet between 350 μm and 450 μm.

Unless the context clearly indicates otherwise, throughout the description and the claims, the words "comprise", "comprising", and the like are to be taken into as an inclusive sense as opposed to an exclusive or exhaustive sense, that is to say, in the sense of "including, but not limited to".

The inventors of the invention have surprisingly found that it is possible to formulate a stable pharmaceutical composition of vildagliptin or pharmaceutically acceptable salt thereof, which can exhibit superior chemical and physical stability by direct compression, and a manufacturing process that is easy and cost effective.

The term "vildagliptin" used throughout the specification refers to not only vildagliptin per se, but also various pharmaceutically acceptable salts and pharmaceutically acceptable hydrates thereof.

The following Examples illustrate aspects of the present invention but are not in any way limiting the scope of invention.

EXAMPLES

Reference Example 1

The main objective is to develop a robust and stable immediate release tablet comprising vildagliptin. The inventors first wanted to decide on the most appropriate manufacturing process. Therefore two different manufacturing processes were tested, dry mixing and wet granulation. All the excipients were selected with the perspective to enhance dissolution, physicochemical characteristics and stability of the drug substance in the final dosage form.

Common diluents such as lactose anhydrous, microcrystalline cellulose, mannitol SD were tested throughout the formulation trials in order to enhance flowability and compaction properties of the powder blend and the most proper ones were finally used in the formulation. Croscarmellose sodium, crospovidone and sodium starch glycolate were tested as disintegrants which provide the necessary force to rapture and eventually disintegrate the tablets. Specifically, due to their large swelling capacity in aqueous solution they enhance the force needed to push particles apart within tablet pores exerted by the water, resulting in rapid tablet disintegration. Magnesium stearate was used in formulations as lubricant to prevent any sticking during compression. The two compositions tested with the two different manufacturing processes are shown in the table below.

Table 1. Compositions prepared with direct compression (composition 1) and wet granulation (composition 2).

|  | Composition 1 | Composition 2 |
|---|---|---|
| Ingredients | % Release | % Release |
| **Internal phase** | | |
| Vildagliptin | 25.00 | 25.00 |
| Microcrystalline cellulose PH102 | 47.84 | 47.84 |
| Lactose anhydrous | 23.91 | 23.91 |
| Sodium starch glycolate | 2.00 | 2.00 |
| Water | - | qs |
| **External phase** | | |
| Magnesium stearate | 1.25 | 1.25 |

Composition 1 was prepared by dry mixing the internal phase excipients for 20 minutes and adding the external phase excipient and mixing for 3 minutes and the resulting tablets showed good characteristics with disintegration time of 3'01"±30" (target disintegration time 1'30"-5;00") and hardness of 105±5 N (target hardness 80-150N). Composition 2 was prepared with wet granulation the internal phase excipients with water and drying the resulting granules and mixing the external phase excipient for 3 minutes and the tablets showed poor characteristics with disintegration time of 11'34±50" (target disintegration time 1'30" - 5;00") and hardness of 203±10N (target hardness 80-150N).

Furthermore both Compositions 1 and 2 were compared against the dissolution profiles of Galvus® (Novartis). The following Table 2 shows the difference in the dissolution profile of Composition 1 prepared by dry mixing and Composition 2 prepared by wet granulation against the dissolution profile of the originators product as percentage of dissolution over time shown in minutes.

Table 2. Dissolution profiles of compositions 1 and 2 and Galvus® shown as percentage of dissolution over time shown in minutes.

| t (min) | Comp 1 % Release | Comp 2 % Release | Galvus® % Release |
|---|---|---|---|
| 5 | 58.30 | 11.85 | 64.80 |
| 10 | 85.34 | 26.21 | 90.74 |
| 15 | 89.85 | 44.17 | 93.84 |
| 30 | 92.95 | 85.12 | 96.68 |

(continued)

| t (min) | Comp 1 % Release | Comp 2 % Release | Galvus® % Release |
|---|---|---|---|
| **45** | 94.39 | 98.41 | 98.04 |
| **60** | 95.44 | 100.67 | 98.94 |

The stability studies for both compositions shown in Tables 3 and 4 revealed that Composition 1 is rather stable during the 6 months period, while Composition 2 exhibited high impurity levels over the specified limits. Moreover, Composition 1 exhibits the best behavior compared to the originator and according to the target characteristics, hence the dry mixing process (direct compression) was selected for the next formulation trials.

Table 3. Stability studies for Composition 1

| **Composition 1** | Limit | Zero time | 25°C 6months | 30°C 6months | 40°C 6months |
|---|---|---|---|---|---|
| **Total Unknown Impurities** | | 0.16% | 0.33% | 0.34% | 0.49% |
| **Impurity F** | 0.2% | 0.03% | 0.11 % | 0.10% | 0.14% |
| **Total Impurities** | 1.0% | 0.19% | 0.44% | 0.44% | 0.63% |

Table 4. Stability studies for Composition 2

| **Composition 2** | Limit | Zero time | 25°C 6months | 30°C 6months | 40°C 6months |
|---|---|---|---|---|---|
| **Total Unknown Impurities** | | 0.18% | 0.39% | 0.43% | 0.56% |
| **Impurity F** | 0.2% | 0.17% | 0.32% | 0.29% | 0.33% |
| **Total Impurities** | 1.0% | 0.35% | 0.74% | 0.72% | 0.89% |

Reference Example 2

For the development of further formulation trials the Design of Experiments (DoE) methodology was applied by the inventors. An early screening DoE based on the principles of the mixture design approach was implemented. An important type of DoE application refers to the preparation and modification of mixtures. For the experiments, microcrystalline cellulose (MCC), lactose anhydrous and sodium starch glycolate were used as typical excipients with diluent and disintegrant properties respectively, while Starch 1500 was added because of its disintegration/diluent properties. The proportions of the incorporated excipients were selected as the main factors for the mixture design, while hardness, disintegration time and dissolution profiles as responses. The constituents were mixed according to mixture design with multiple constraints on the component proportions, using fixed intervals as stated in Tables 5 and 6.

Table 5. Factors and intervals selected to perform the mixture design

| Factors in total internal phase excipients | Level | |
|---|---|---|
| | **Low** | **High** |
| $X_1$ = percentage of MCC (%) | 10.0 | 43.2 |
| $X_2$ = percentage of Lactose anhydrous (%) | 10.0 | 43.2 |
| $X_3$ = percentage of Sodium starch glycolate (%) | 2.0 | 7.0 |
| $X_4$ = percentage of Starch 1500 (%) | 13.5 | 20.0 |

Table 6. DoE parameter settings including ingredients and proportion in the mixture of excipients

| Ru n | MCC ($X_1$) | Lactose anhydrous ($X_2$) | Sodium starch glycolate ($X_3$) | Starch 1500 ($X_4$) |
|---|---|---|---|---|
| 3 | 10 | 43 | 2 | 19 |

(continued)

| Ru n | MCC ($X_1$) | Lactose anhydrous ($X_2$) | Sodium starch glycolate ($X_3$) | Starch 1500 ($X_4$) |
|------|-------------|---------------------------|----------------------------------|----------------------|
| 4 | 43 | 10 | 2 | 19 |
| 5 | 10 | 42 | 2 | 20 |
| 6 | 26 | 26 | 5 | 17 |
| 7 | 43 | 10 | 7 | 14 |
| 8 | 18 | 35 | 3 | 18 |
| 9 | 18 | 35 | 6 | 15 |
| 10 | 18 | 34 | 6 | 16 |
| 11 | 18 | 34 | 3 | 18 |
| 12 | 34 | 18 | 6 | 16 |
| 13 | 35 | 18 | 6 | 15 |
| 14 | 42 | 10 | 2 | 20 |
| 15 | 34 | 18 | 3 | 18 |
| 16 | 35 | 18 | 3 | 18 |
| 17 | 10 | 43 | 7 | 14 |
| 18 | 10 | 42 | 7 | 15 |
| 19 | 42 | 10 | 7 | 15 |

The design points were generated by Minitab® statistical software, while the amounts of Vildagliptin and magnesium stearate per tablet (200 mg) were fixed at 50.0 and 2.50 mg, respectively.

Table 7 gathers the measured experimental data of the Vildagliptin tablet disintegration time, hardness and drug release rate at the 10 min of dissolution time. The flowability of each composition powder bulk was measured by the Carr's index and varied from 24 to 32, a fact that underlies a poor flowability.

Table 7. Average tablet hardness, disintegration time and percentage of drug dissolved in the first 10 minutes

| Formulation | Disintegration (s) | Hardness (N) | Dissolution (%) |
|-------------|--------------------|--------------| ----------------|
| Comp 3 | 2'14" | 70 | 88.49 |
| Comp 4 | 5'51" | 152 | 68.54 |
| Comp 5 | 3'16" | 74 | 89.56 |
| Comp 6 | 5'29" | 101 | 71.40 |
| Comp 7 | 4'23" | 151 | 70.32 |
| Comp 8 | 4'28" | 90 | 88.97 |
| Comp 9 | 3'49" | 84 | 96.10 |
| Comp 10 | 3'4" | 85 | 92.57 |
| Comp 11 | 4'22" | 88 | 63.02 |
| Comp 12 | 4'10" | 119 | 69.83 |
| Comp 13 | 5'10" | 133 | 69.06 |
| Comp 14 | 6'04" | 157 | 60.75 |
| Comp 15 | 5'54" | 130 | 69.87 |
| Comp 16 | 6'09" | 144 | 60.28 |
| Comp 17 | 3'01" | 73 | 93.15 |

(continued)

| Formulation | Disintegration (s) | Hardness (N) | Dissolution (%) |
|---|---|---|---|
| Comp 18 | 2'51" | 74 | 91.10 |
| Comp 19 | 4'32" | 174.33 | 70.01 |

Generally, linear model equation is the most appropriate to the mixture design since the relations between the ingredients of mixture are showed by directly proportional responses. In the present case, the linear model obtained from the regression data for tablet hardness (HRDS) is the following:

$$HRDS = 873.132X_1 + 548.686X_2 - 9349.78X_3 - 1185.17X_4 \tag{1}$$

Accordingly, linear model equations for disintegration time (DISNT) and Vildagliptin release rate (VRR) calculated from the regression data are as follows:

$$DISNT = -415.415X_1 - 854.445X_2 + 8273.34X_3 + 2684.80X_4 \tag{2}$$

and

$$VRR = 205.610X_1 + 284.821X_2 - 642.225X_3 - 241.663X_4 \tag{3}$$

The results show that compositions 3, 9 and 17 with various Starch 1500 percentages ranging from 2 to 7% and lactose anhydrous percentage greater than MCC, showed the best behaviour with disintegration time and drug release rate within the optimized design space of the mixture design. Specifically formulation 9 exhibited a typical hardness value (~ 87 N), while disintegration time (~4 min) is more than adequate for an immediate release drug formulation. On the other hand, formulations 6, 13 and 14 with lactose anhydrous percentage equal or lesser than the respective MCC exhibited better hardness in combination with optimum disintegration time. The resulted design space graph led to further optimization and prediction of a new experiment.

Example 3

For the preparation of a second DoE different disintegrants (except Starch 1500) and diluents were tested. Therefore, the main idea was to keep MCC as the standard diluent throughout the trials while lactose anhydrous, mannitol SD, croscarmellose sodium and crospovidone were studied quantitatively and qualitatively as second diluent and disintegrant respectively. For that, two numerical factors such as the ratio of the second diluent over MCC and the percentage of the disintegrant were variated. The methodology for this DoE was the full factorial design that included 2 center points for a total of 24 runs. Likely operating ranges of the independent variables were established, and experimental ranges (boundaries) within these operating ranges were chosen (Table 8). The factors that were selected for this DoE with the respective constraints are presented in Table 8 and Table 9. Only the compositions comprising 7-9% by weight of disintegrant are according to the invention.

Table 8. Factors selected to perform factorial design

| Factors in total internal phase excipients | Level | |
|---|---|---|
| | Low | High |
| $X_1$ = fraction of Diluent2/MCC | 0.25 | 4.00 |
| $X_2$ = percentage of Disintegrant (%) | 2.0 | 7.0 |
| $X_3$ = type of Diluent2 | Lactose anhydrous | Mannitol SD |
| $X_4$ = type of Disintegrant | Crospovidone | Croscarmellose sodium |

Table 9. DoE parameter settings including ingredients, proportion and type of excipients

| Run | Diluent2/MCC ($X_1$) | Disintegrant (%) ($X_2$) | Diluent2 ($X_3$) | Disintegrant ($X_1$) |
|---|---|---|---|---|
| 20 | 0.25 | 0.07 | Mannitol SD | Crospovidone |
| 21 | 0.25 | 0.07 | Lactose Anhydrous | Croscarmellose sodium |
| 22 | 4 | 0.02 | Lactose Anhydrous | Crospovidone |
| 23 | 2.125 | 0.045 | Mannitol SD | Crospovidone |
| 24 | 4 | 0.07 | Mannitol SD | Crospovidone |
| 25 | 2.125 | 0.045 | Lactose Anhydrous | Crospovidone |
| 26 | 0.47 | 0.045 | Lactose Anhydrous | Crospovidone |
| 27 | 0.47 | 0.045 | Mannitol SD | Crospovidone |
| 28 | 0.25 | 0.02 | Mannitol SD | Croscarmellose sodium |
| 29 | 4 | 0.07 | Lactose Anhydrous | Croscarmellose sodium |
| 30 | 0.25 | 0.02 | Lactose Anhydrous | Croscarmellose sodium |
| 31 | 4 | 0.07 | Lactose Anhydrous | Crospovidone |
| 32 | 4 | 0.02 | Mannitol SD | Croscarmellose sodium |
| 33 | 4 | 0.02 | Lactose Anhydrous | Croscarmellose sodium |
| 34 | 4 | 0.07 | Mannitol SD | Croscarmellose sodium |
| 35 | 2.125 | 0.045 | Mannitol SD | Croscarmellose sodium |
| 36 | 0.25 | 0.02 | Mannitol SD | Crospovidone |
| 37 | 4 | 0.02 | Mannitol SD | Crospovidone |
| 38 | 2.125 | 0.045 | Lactose Anhydrous | Croscarmellose sodium |
| 39 | 0.25 | 0.07 | Lactose Anhydrous | Crospovidone |
| 40 | 0.25 | 0.02 | Lactose Anhydrous | Crospovidone |
| 41 | 0.47 | 0.045 | Mannitol SD | Croscarmellose sodium |
| 42 | 0.47 | 0.045 | Lactose Anhydrous | Croscarmellose sodium |
| 43 | 0.25 | 0.07 | Mannitol SD | Croscarmellose sodium |

The design points were generated by Minitab® statistical software and the respective tablet formulations are based upon Table 8 and Table 9 with a fixed final tablet weight of 200 mg. A summarization of the DoE experimental results regarding the pharmacotechnical characterization of the tablets are presented in Table 10. Dissolution of the Vildagliptin tablets was measured according to USP 2 apparatus, at a paddle speed of 50 rpm, in 500 mL of pH 1.2 buffer solution at $37 \pm 0.5°C$. Additionally, Carr's indices have not been included in the factorial responses as in all formulations found to be in the range of 20-25% with no extreme differentiations.

Table 10. Average tablet hardness and disintegration time

| Formulation | Disintegration (s) | Hardness (N) |
|---|---|---|
| Comp 20 | 2'02" | 133.44 |
| Comp 21 | 3'43" | 141.00 |
| Comp 22 | 2'50" | 71.50 |
| Comp 23 | 42" | 69.00 |
| Comp 24 | 28" | 87.10 |
| Comp 25 | 5'09" | 88.60 |

(continued)

| Formulation | Disintegration (s) | Hardness (N) |
|---|---|---|
| Comp 26 | 5'55" | 112.97 |
| Comp 27 | 1'12" | 76.00 |
| Comp 28 | 1'22" | 95.89 |
| Comp 29 | 3'21" | 63.05 |
| Comp 30 | 4'05" | 122.95 |
| Comp 31 | 2'58" | 77.35 |
| Comp 32 | 40" | 78.19 |
| Comp 33 | 4'20" | 89.05 |
| Comp 34 | 41" | 71.05 |
| Comp 35 | 39" | 70.85 |
| Comp 36 | 1'28" | 88.00 |
| Comp 37 | 40" | 81.50 |
| Comp 38 | 4'46" | 92.10 |
| Comp 39 | 3'30" | 131.69 |
| Comp 40 | 3'40" | 124.01 |
| Comp 41 | 1'10" | 98.37 |
| Comp 42 | 4'40" | 123.58 |
| Comp 43 | 1'41" | 103.57 |

An empirical mathematical model was fitted for each response variable. Eqns. 4 and 5 show the model for the response variable HRDS (Hardness) and DISNT (Disintegration), respectively. These regression models include terms representing the effect of each input variable ($X_1$, $X_3$), two-way interactions ($X_1 X_3$ and $X_3 X_4$), and the random experimental error.

$$HRDS = -3.80X_1 - 15.64X_3 + 4.20X_1X_3 - 94.03 \tag{4}$$

$$DISNT = -19.69X_1 - 94.50X_3 + 16.8X_4 + 4.15X_1X_3 - 11.76X_3X_4 + 184.8 \tag{5}$$

The target characteristics for the response optimization are set to 120 N for hardness and ~3min for disintegration time. The composite desirability of 0.9448 is a very good score and indicates that all responses were close to their ideal settings. A formulation that is based upon the optimal responses has the following values for $X_1$, $X_2$, $X_3$ and $X_4$.

| $X_1$ | $X_2$ | $X_3$ | $X_4$ |
|---|---|---|---|
| 0.78 | 6% | Lactose anhydrous | Croscarmellose sodium |

Reference Example 4

Taken together the data from the optimization studies led the inventors to test 2 more compositions, one with croscarmellose sodium and one with crospovidone as a disintegrant both at a percentage of 6%. The compositions are shown in Table 11 below.

Both compositions were prepared by dry mixing the internal phase excipients for 20 minutes and then mixing the external phase excipient for 3 minutes. The resulting tablets showed the good characteristics with disintegration time for composition 3 of 5'46"±30" (target disintegration time 1'30"- 5'00") and hardness of 178±9 N (target hardness 80-150N) and a Carr's index of 20%. Composition 4 showed poor characteristics with disintegration time of 7'55±50" (target disintegration time 1'30"- 5'00") and hardness of 170±5N (target hardness 80-150N) and a Carr's index of 21%. Both compositions had hardness above the specified limits, Carr's index was calculated at about 20% for both, indicating smooth compression procedure. However, the dissolution profile showed a faster release rate for composition 3 during the time of the test and therefore composition was chosen for further optimization.

Table 11. Quantitative and qualitative formulations for Composition 44 and Composition 45

|  | Composition 44 | Composition 45 |
|---|---|---|
| Ingredients | % | % |
| **Internal phase** |  |  |
| Vildagliptin | 25.00 | 25.00 |
| Microcrystalline cellulose PH102 | 45.17 | 47.84 |
| Lactose anhydrous | 22.58 | 23.91 |
| Croscarmellose sodium | 6.00 | - |
| Crospovidone | - | 6.00 |
| **External phase** |  |  |
| Magnesium stearate | 1.25 | 1.25 |
| **Total** | **100.00** | **100.00** |

Example 5

The inventors continued the optimization process by trying to improve the physical properties of the vildagliptin tablets and therefore increased the croscarmellose sodium percentage of composition 46 from 6% to 8% and at the same time decreasing the percentage of magnesium stearate. The resulting composition is shown in the table below.

Table 12. Quantitative and qualitative formulations for Composition 46

|  | Composition 46 |
|---|---|
| Ingredients | % |
| **Internal phase** |  |
| Vildagliptin | 25.00 |
| Microcrystalline cellulose PH102 | 44.33 |
| Lactose anhydrous | 21.92 |
| Croscarmellose sodium | 8.00 |
| **External phase** |  |
| Magnesium stearate | 0.75 |
| **Total** | **100.00** |

Composition 46 was prepared by dry mixing the internal phase excipients for 20 minutes and adding the external phase excipient and mixing for 3 minutes and the resulting tablets showed good characteristics with disintegration time of 2'38"±30" (target disintegration time 1'30"-5;00") and hardness of 140±20N (target hardness 80-150N) and Carr's index of 21%. The 6 months stability data for the final composition 46 are listed in Table 13.

Table 13. 6 months stability data for Comp 46

| Comp 46 | Limit | Zero time | 25°C | 30°C | 40°C |
|---|---|---|---|---|---|
| **Total Unknown Impurities** |  | 0.23% | 0.28% | 0.31% | 0.40% |
| **Impurity F** | 0.2% | 0.05% | 0.07% | 0.07% | 0.12% |

(continued)

| Comp 46 | Limit | Zero time | 25°C | 30°C | 40°C |
|---|---|---|---|---|---|
| **Total Impurities** | 1.0% | 0.28% | 0.35% | 0.38% | 0.52% |

Example 6

The final optimization step of the development incorporated two more compositions with two different particle size distributions of the same Vildagliptin powder, in order to finalize the formulation and to examine any uniformity issues. For comparison reasons, Trials 46.1 and 46.2 were prepared with the same percentages of the ingredients as in Composition 46. The following table (Table 14) depicts the average assay uniformity values of the blend of the final mixture, wherein Trial 46.1 Vildagliptin powder has D(90) above 750 $\mu$m (not according to the invention) and in Trial 46.2 Vildagliptin powder has D(90) in the range of 350-450 $\mu$m as measured by laser light diffraction (according to the invention).

Table 14. Assay uniformity for Trial 46.1 and Trial 46.2

| Trial 46.1 | | Trial 46.2 | |
|---|---|---|---|
| Sample 1 | 109.1 | Sample 1 | 99.7 |
| Sample 2 | 92.6 | Sample 2 | 100.4 |
| Sample 3 | 121.1 | Sample 3 | 98.7 |
| Sample 4 | 105.9 | Sample 4 | 98.9 |
| Sample 5 | 94.0 | Sample 5 | 97.9 |
| Sample 6 | 110.2 | Sample 6 | 99.3 |
| **Average** | **105.5** | **Average** | **99.2** |
| **% RSD** | **10.19** | **% RSD** | **0.87** |

Clearly, PSD plays a significant role in the uniformity of the blend of the final mixture and as a result Trial 46.2 showed the best physical properties during the lab manufacturing process.

Furthermore, Vildagliptin in formulation 46.2 remains in the same crystalline form after the manufacturing procedure and during stability period of 6 months as it was verified by the XRD patterns.

**Claims**

1.  A pharmaceutical composition comprising Vildagliptin or a pharmaceutically acceptable salt thereof as the pharmaceutically active ingredient and at least one disintegrant, wherein 90% of the particles by volume of the active pharmaceutical ingredient is between 350 and 450$\mu$m as measured by laser light diffraction, wherein the amount of the disintegrant is from 7 to 9 % by weight of the composition.

2.  The pharmaceutical composition according to claim 1 wherein the disintegrant is croscarmellose sodium.

3.  The pharmaceutical composition according to any of the preceding claims wherein it further comprises an amount of diluent or combination of diluents between 50 and 90% by weight, preferably from 60 to 80% by weight of the composition.

4.  The pharmaceutical composition according to any of preceding claims, wherein the weight ratio between diluent or combination of diluents and disintegrant is from 5:1, preferably from 7.5:1, more preferably is 10:1.

5.  The pharmaceutical composition according to any of preceding claims, wherein the diluent is a combination of lactose and microcrystalline cellulose.

6.  The pharmaceutical composition according to any of the preceding claims, wherein it further comprises magnesium stearate.

7. A process for the preparation of a pharmaceutical composition according to claim 1 comprising the steps of:

- Mixing Vildagliptin or a pharmaceutically acceptable salt thereof having 90% of the particles by volume between 350 and 450 $\mu$m as measured by laser light diffraction with at least one disintegrant in the amount of from 7 to 9 % by weight of the composition and optionally other pharmaceutically acceptable excipient until a uniform blend is reached;
- Adding to the above mixture a pharmaceutically acceptable glidant and mixing until uniformity of blend;
- Compressing the resulting mixture into tablets;
- Optionally coating the tablets.

8. The process for the preparation of a pharmaceutical composition according to claim 7 wherein the disintegrant is croscarmellose sodium.

**Patentansprüche**

1. Pharmazeutische Zusammensetzung bestehend aus Vildagliptin oder einem pharmazeutisch verwendbaren Salz davon als pharmazeutischen Wirkstoff sowie aus mindestens einem Sprengmittel, **dadurch gekennzeichnet, dass** die Partikelgröße der Partikel, die ein Volumenverhältnis von 90% des Gesamtvolumens des pharmazeutischen Wirkstoffs ausmachen, zwischen 350 und 450 $\mu$m liegt, wie diese mittels Laser-Streulichtspektrometers gemessen wurde, wobei das Gewichtsverhältnis des Sprengmittels in der Zusammensetzung zwischen 7% und 9% liegt.

2. Pharmazeutische Zusammensetzung nach Anspruch 1, **dadurch gekennzeichnet, dass** das Sprengmittel Croscarmellose-Natrium ist.

3. Pharmazeutische Zusammensetzung nach einem der vorhergehenden Ansprüche, **dadurch gekennzeichnet, dass** sie auch ein Verdünnungsmittel oder eine Kombination von Verdünnungsmitteln in einem Gewichtsverhältnis zwischen 50 und 90%, vorzugsweise zwischen 60 und 80%, enthält.

4. Pharmazeutische Zusammensetzung nach einem der vorhergehenden Ansprüche, **dadurch gekennzeichnet, dass** das Gewichtsverhältnis vom Verdünnungsmittel bzw. von der Kombination der Verdünnungsmittel zum Sprengmittel 5:1, vorzugsweise 7,5:1 und noch bevorzugter 10:1 ist.

5. Pharmazeutische Zusammensetzung nach einem der vorhergehenden Ansprüche, **dadurch gekennzeichnet, dass** das Verdünnungsmittel eine Kombination von Lactose und Microcrystalline Cellulose ist.

6. Pharmazeutische Zusammensetzung nach einem der vorhergehenden Ansprüche, **dadurch gekennzeichnet, dass** sie auch Magnesiumstearat enthält.

7. Verfahren für die Zubereitung einer pharmazeutischen Zusammensetzung nach Anspruch 1, welches aus den nachstehenden Schritten besteht:

- Vildagliptin oder einen pharmazeutisch verwendbaren Salz davon mit einer Partikelgröße zwischen 350 und 450 $\mu$m, wie diese mittels Laser-Streulichtspektrometers gemessen wurde, die ein Volumenverhältnis von 90% des Gesamtvolumens des pharmazeutischen Wirkstoffs ausmachen, mit mindestens einem Sprengmittel mischen, dessen Gewichtsverhältnis in der Zusammensetzung zwischen 7% und 9% liegt, und wahlweise auch mit weiteren pharmazeutisch verwendbaren Hilfsstoffen mischen, bis eine einheitliche Mischung erreicht wird.
- Ein pharmazeutisch verwendbares Gleitmittel in die obere Mischung hinzufügen und rühren bis die Mischung einheitlich wird;
- Die daraus resultierende Mischung tablettieren;
- Optional die Tabletten mit Film überziehen.

8. Verfahren für die Zubereitung einer pharmazeutischen Zusammensetzung nach Anspruch 7, **dadurch gekennzeichnet, dass** das Sprengmittel Croscarmellose-Natrium ist.

**Revendications**

1. Composition pharmaceutique comprenant de la vildagliptine ou un sel pharmaceutiquement acceptable de celle-ci en tant qu'ingrédient pharmaceutiquement actif et au moins un désintégrant, dans lequel 90 % des particules en volume de l'ingrédient pharmaceutique actif sont comprises entre 350 et 450 $\mu$m telles que mesurées par diffraction de lumière laser, et dans lequel la quantité de désintégrant est de 7 à 9 % en poids de la composition.

2. Composition pharmaceutique selon la 1ère revendication, dans laquelle le désintégrant est le croscarmellose sodique.

3. Composition pharmaceutique selon l'une quelconque des revendications précédentes, qui comprend en outre une quantité de diluant ou de combinaison de diluants comprise entre 50 et 90 % en poids, de préférence de 60 à 80 % en poids de la composition.

4. Composition pharmaceutique selon l'une quelconque des revendications précédentes, dans laquelle le rapport en poids entre le diluant ou la combinaison de diluants et le désintégrant est de 5:1, de préférence de 7,5:1, mieux encore de 10: 1.

5. Composition pharmaceutique selon l'une quelconque des revendications précédentes, dans laquelle le diluant est une combinaison de lactose et de cellulose microcristalline.

6. Composition pharmaceutique selon l'une quelconque des revendications précédentes, qui comprend en outre du stéarate de magnésium.

7. Procédé de préparation d'une composition pharmaceutique selon la 1ère revendication, qui comprend les étapes suivantes :

   - Le mélange de la vildagliptine ou un sel pharmaceutiquement acceptable de celle-ci avec 90 % des particules en volume entre 350 et 450 $\mu$m telles que mesurées par diffraction de la lumière laser, avec au moins un désintégrant à raison de 7 à 9 % en poids de la composition et éventuellement un autre excipient pharmaceutiquement acceptable jusqu'à l'homogénéité complète ;
   - L'ajout au mélange ci-dessus d'un glissant pharmaceutiquement acceptable et le mélange jusqu'à l'homogénéité complète ;
   - La compression du mélange résultant en comprimés ;
   - Enrobage facultatif des comprimés.

8. Procédé de préparation d'une composition pharmaceutique selon la 7ème revendication, dans lequel l'agent désintégrant est le croscarmellose sodique.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6166063 A
- EP 2468361 A
- EP 1841413 A
- EP 2165703 A

**Non-patent literature cited in the description**

- Handbook of pharmaceutical excipients. American Pharmaceutical Association
- Handbook of Pharmaceutical Excipients. Science and